# EUROPEAN PATENT SPECIFICATION

(11) **EP 0 738 273 B1**
(45) Date of publication and mention of the grant of the patent: **17.11.1999**
(21) Application number: 95907232.3
(22) Date of filing: 13.12.1994
(51) Int. Cl.: C07H 19/06, A61K 31/70

(54) **MONOHYDRATE OF (E)-2'-DEOXY-2'-(FLUOROMETHYLENE)CYTIDINE**
(E)-2'-DEOXY-2'-(FLUORMETHYLEN)CYTIDIN-MONOHYDRAT
MONOHYDRATE DE (E)-2'-DESOXY-2'-(FLUOROMETHYLENE)CYTIDINE

(30) Priority: 07.01.1994 US 178952
(43) Date of publication of application: 23.10.1996
(73) Proprietor: MERRELL PHARMACEUTICALS INC., Cincinnati, Ohio 45215-6300 (US)
(72) Inventor: DONALDSON, Richard, E., Midland, MI 48642 (US)
(74) Representative: VOSSIUS & PARTNER
(86) International application number: US9414648
(87) International publication number: WO9518815

(56) References cited:
- EP-A- 0 372 268
- EP-A- 0 398 230
- EP-A- 0 443 471
- WO-A-93/23414

## Description

### BACKGROUND OF THE INVENTION

The present invention relates to the novel monohydrate of (E)-2'-deoxy-2'-(fluoromethylene)cytidine useful as a ribonucleotide reductase inhibitor in the treatment of patients afflicted with neoplastic or viral disease states. McCarthy et al. discloses related compounds which are certain novel 2'-halomethylidine, 2'-ethenylidine and 2'-ethynyl cytidine, uridine and guanosine derivatives and compositions thereof which are useful in the treatment of patients afflicted with neoplastic or viral diseases, in European Patent Application Publication No. 0 372 268 published June 13, 1990.

### SUMMARY OF THE INVENTION

The present invention provides the novel compound (E)-2'-deoxy-2'-(fluoromethylene)cytidine monohydrate of the formula; 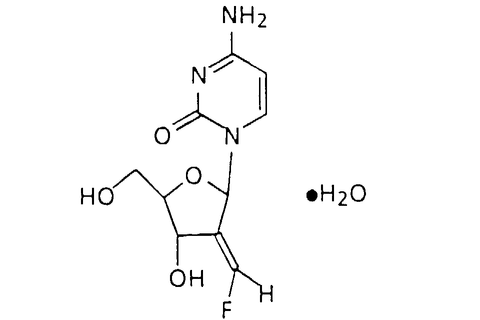 which is useful as a ribonucleotide reductase inhibitor in the treatment of patients afflicted with neoplastic or viral disease states. The above monohydrate has beneficial manufacturing characteristics over the anhydrous form of (E)-2'-deoxy-2'-(fluoromethylene)cytidine.

The present invention provides a novel process for preparing the above monohydrate
1.
   (a) reacting a compound of the formula 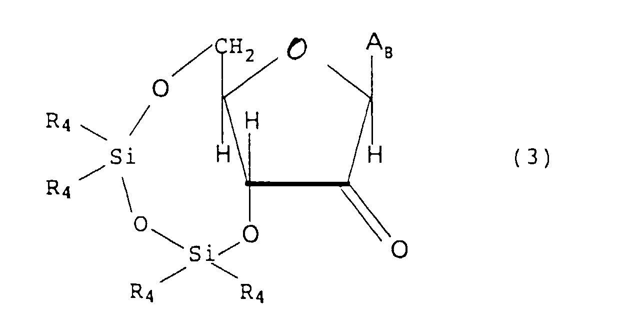 wherein R₄ is C₂-C₇ alkyl or C₅-C₇ cycloalkyl and A_{B} is a radical of the formula 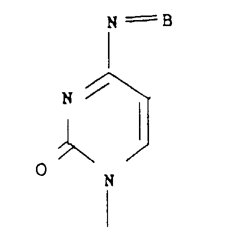 and B is a suitable nitrogen blocking group of the formula CR₁NR₂R₃ wherein R₁ is a hydrogen or C₁-C₄ alkyl and R₂ and R₃ are independently C₁-C₄ alkyl, with a suitable phosphonate ylid of the formula (X)₂OP=CF(SO₂Ar) wherein Ar is an aryl group and X is a phenoxy or C₁-C₄ alkoxy to produce an exocyclic fluorovinyl sulfone;
   (b) reacting the exocyclic fluorovinyl sulfone with a suitable base or suitable weak acid to produce a deprotected amine;
   (c) reacting the deprotected amine with a stannylating reagent of the formula (R)₃SnH wherein R is aryl or C₁-C₄ alkyl to produce an exocyclic (fluorovinyl)stannane;
   (d) reacting the exocyclic (fluorovinyl)stannane with a protolysis agent and, either concomitantly or sequentially, reacting the silyl protecting group with a suitable acid or a fluoride ion source.
   or
2.
   (a) reacting a compound of the formula 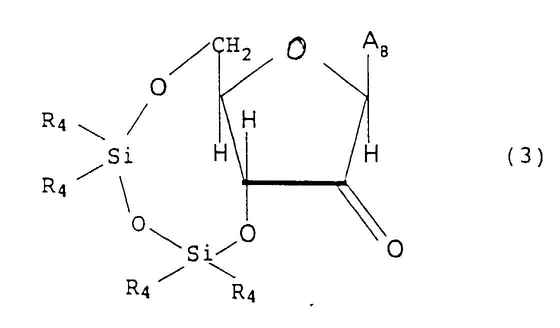 wherein R₄ is C₂-C₇ alkyl or C₅-C₇ cycloalkyl and A_{B} is a radical of the formula 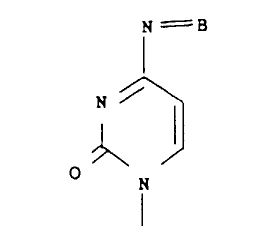 and B is a suitable nitrogen blocking group of the formula CR₁NR₂R₃ wherein R₁ is a hydrogen or C₁-C₄ alkyl and R₂ and R₃ are independently C₁-C₄ alkyl, with a suitable base or suitable weak acid to produce a deprotected amine;
   (b) reacting the deprotected amine with a suitable phosphonate ylid of the formula (X)₂OP=CF(SO₂Ar) wherein Ar is an aryl group and X is a phenoxy or C₁-C₄ alkoxy to produce an exocyclic fluorovinyl sulfone;
   (c) reacting the exocyclic fluorovinyl sulfone with a stannylating reagent of the formula (R)₃SnH wherein R is aryl or C₁-C₄ alkyl to produce an exocyclic (fluorovinyl)stannane;
   (d) reacting the exocyclic (fluorovinyl)stannane with a protolysis agent and, either concomitantly or sequentially, reacting the silyl protecting group with a suitable acid or a fluoride ion source.
   or
3.
   (a) reacting a compound of the formula 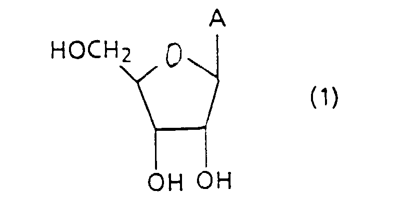 wherein A is a radical of the formula 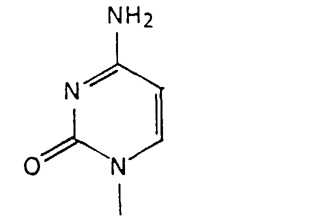 with excess 1,3-dichloro-1,1,3,3-tetraalkyldisiloxane and triethylamine followed by treatment with SO₃-pyridine complex to produce a 3',5'-protected-2'-keto derivative;
   (b) reacting the 3',5'-protected-2'-keto derivative with a suitable phosphonate ylid of the formula (X)₂OP=CF(SO₂Ar) wherein Ar is an aryl group and X is a phenoxy or C₁-C₄ alkoxy to produce an exocyclic fluorovinyl sulfone;
   (c) reacting the exocyclic fluorovinyl sulfone with a stannylating reagent of the formula (R)₃SnH wherein R is aryl or C₁-C₄ alkyl to produce an exocyclic (fluorovinyl)stannane;
   (d) reacting the exocyclic (fluorovinyl)stannane with a protolysis agent and, either concomitantly or sequentially, reacting the silyl protecting group with a suitable acid or a fluoride ion source.

### DETAILED DESCRIPTION OF THE INVENTION

As used herein the term "C₁-C₄ alkyl" refers to a saturated straight or branched chain hydrocarbon radical of one to four carbon atoms. Included within the scope of this term are methyl, ethyl, n-propyl, isopropyl, n-butyl, isobutyl and the like. The term "C₁-C₄ alkoxy" refers to an alkyloxy radical made up of an oxygen radical bearing a saturated straight or branched chain hydrocarbyl radical of one to four carbon atoms and specifically includes methoxy, ethoxy, propyloxy, isopropyloxy, n-butyloxy, isobutyloxy, sec-butyloxy, tertiary butyloxy and the like. As used herein the term "C₂-C₇ alkyl" refers to a saturated straight or branched chain hydrocarbon radical of two to seven carbon atoms. Included within the scope of this term are ethyl, n-propyl, isopropyl, n-butyl, isobutyl, sec-butyl, tert-butyl, pentyl, neopentyl, 2-methylbutyl, 3-methylbutyl, hexyl, heptyl and the like. As used herein the term "C₅-C₇ cycloalkyl" refers to a saturated cyclic hydrocarbon radical of 5 to 7 carbon atoms. Included within the scope of this term are cyclopentyl, cyclohexyl, cycloheptyl and the like. As used herein the term "1,3-dichloro-1,1,3,3-tetraalkyldisiloxane" refers to compounds wherein the alkyl substituents of the 1,3-(1,1,3,3-tetraalkyldisiloxanylidene) are defined by R₄. R₄ is a C₂-C₇ alkyl or C₅-C₇ cycloalkyl substituent. Examples of suitable R₄ substituents are ethyl, n-propyl, isopropyl, n-butyl, isobutyl, sec-butyl, tert-butyl, pentyl, neopentyl, 2-methylbutyl, 3-methylbutyl, hexyl, heptyl, cyclopentyl, cyclohexyl, cycloheptyl and the like. The term "halogen" or "halo" refers to a fluorine, chlorine, bromine, or iodine atom. The term "Ar" or "aryl" refers to an aromatic radical of from about 6 to 12 carbon atoms, such as phenyl, naphthyl or phenyl(C₁-C₄)alkyl groups, wherein said groups are optionally substituted with one, two or three substituents selected from the group consisting of C₁-C₄ alkyl, halo-substituted C₁-C₄ alkyl, halogen or C₁-C₄ alkoxy. The term "phenyl(C₁-C₄)alkyl" refers to a phenyl group substituted with a C₁-C₄ alkyl including phenylmethyl, phenethyl and the like. Specifically included within the scope of the term "Ar" or "aryl" are phenyl, p-toluoyl, p-methoxyphenyl, p-chlorophenyl, naphthyl and the like.

The general synthetic process of the present invention is set forth in Schemes A and AI. All the substituents, unless otherwise indicated, are previously defined. The reagents and starting materials for use in this process are readily available to one of ordinary skill in the art.

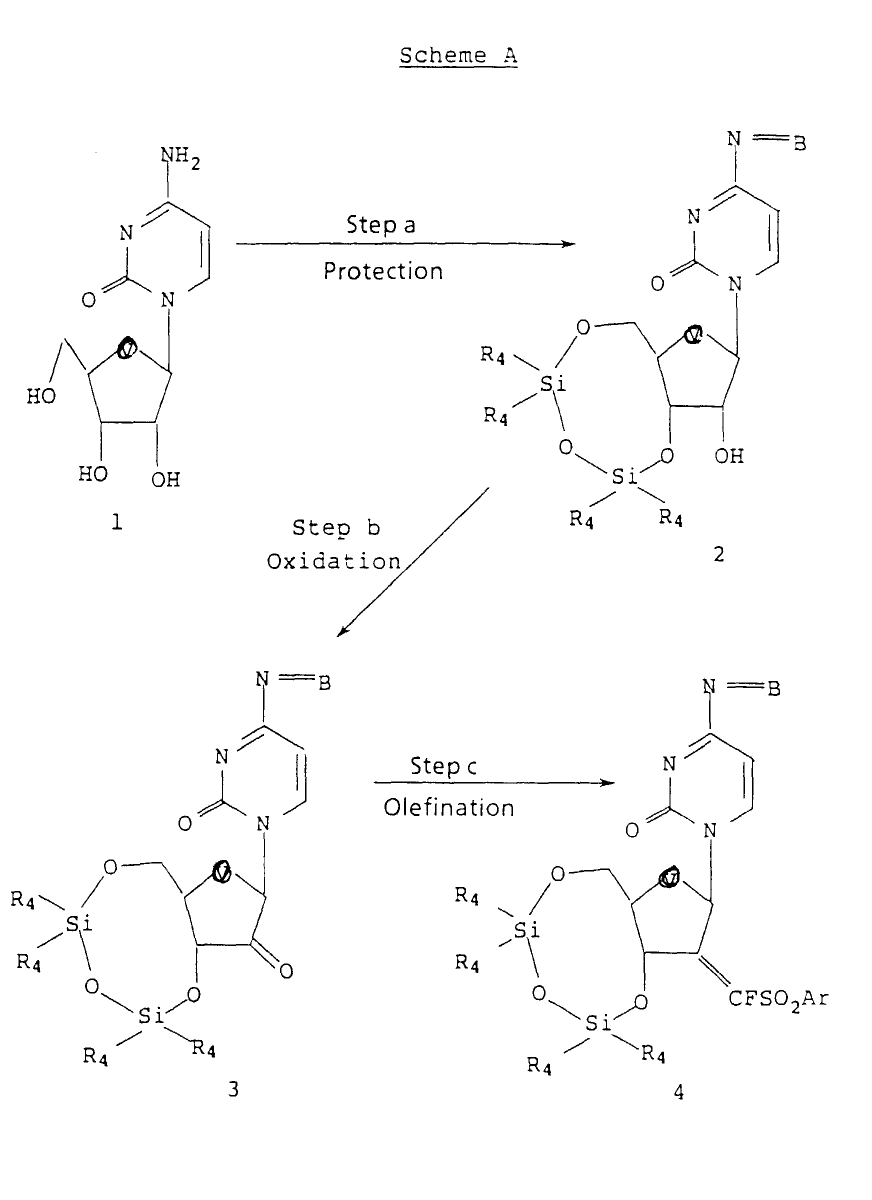

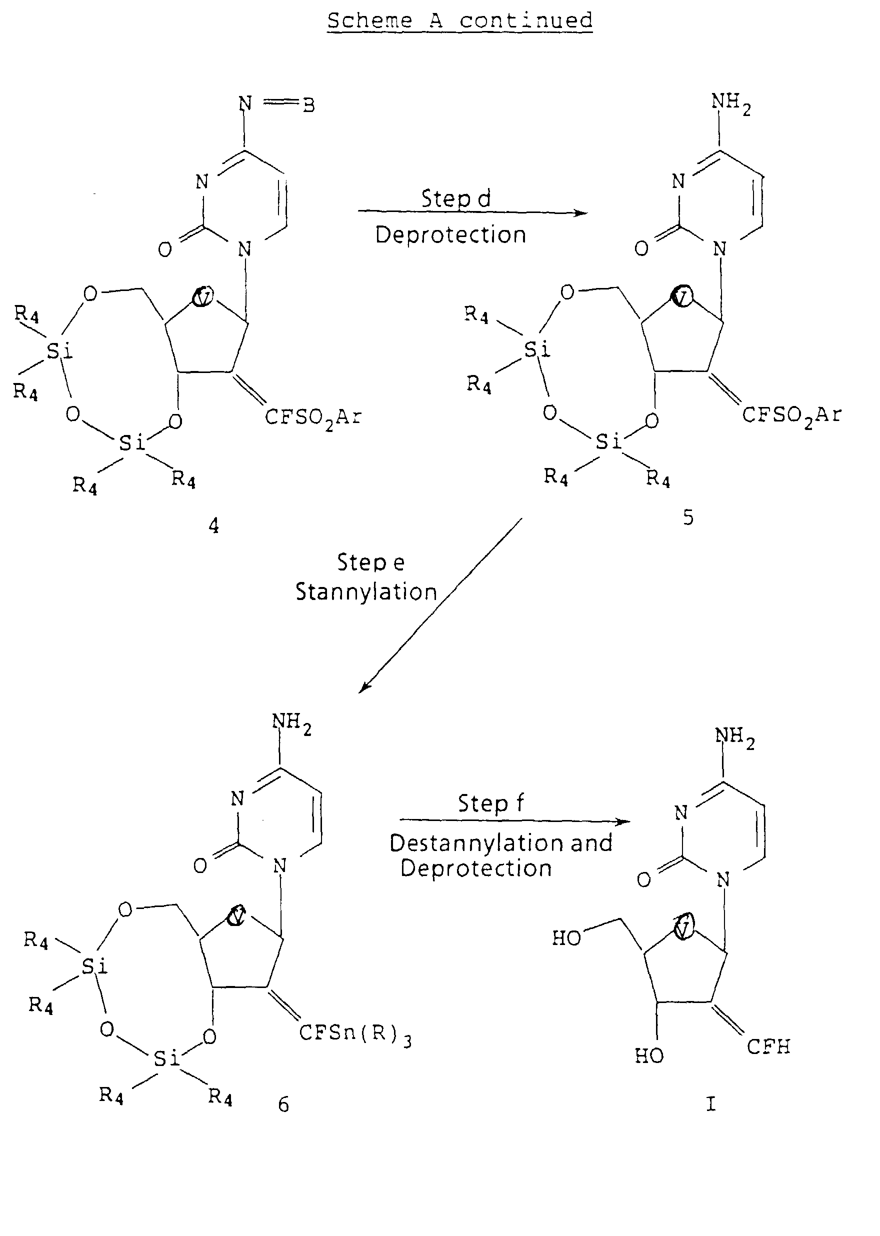

In Scheme A, step a, the 3' and 5' hydroxyls of the appropriately substituted cytidine derivative of structure (1) are protected as the 1,3-(1,1,3,3-tetraalkyldisiloxanylidene) derivative and the amino function is protected with a suitable nitrogen blocking group to provide the compound defined by structure (2). The alkyl substituents of the 1,3-(1,1,3,3-tetraalkyldisiloxanylidene) are defined by R₄. R₄ is a C₂-C₇ alkyl or C₅-C₇ cycloalkyl substituent. Examples of suitable R₄ substituents are ethyl, n-propyl, isopropyl, n-butyl, isobutyl, sec-butyl, tert-butyl, pentyl, neopentyl, 2-methylbutyl, 3-methylbutyl, hexyl, heptyl, cyclopentyl, cyclohexyl, cycloheptyl and the like. The preferred R₄ substituents are isopropyl, isobutyl, cyclopentyl and the like. The most preferred R₄ substituent is isopropyl. A suitable nitrogen blocking group is bound to nitrogen as the imine derivative and is stable to oxidizing conditions such as methyl sulfoxide/oxalyl chloride and to strong organic bases, such as lithium diisopropylamide. For example, suitable nitrogen blocking groups would be N-(N',N'-dimethylaminomethylene)amine, N-(methyl-N',N'-dimethylaminoethylene)amine, N-(methyl-N',N'-diethylaminomethylene)amine, N-(ethyl-N',N'-diethylaminomethylene)amine, and the like. The preferred nitrogen blocking is N-(N',N'-dimethylaminomethylene)amine.

More specifically, in Scheme A, step a; the cytidine derivative (1) is treated with an equivalent of 1,3-dichloro-1,1,3,3-tetraalkyldisiloxane in a basic organic solvent, such as pyridine and allowed to stir for 12 to 24 hours at approximately 10°C to 30°C. The 1,3-dichloro-1,1,3,3-tetraalkyldisiloxane is readily available to one of ordinary skill in the art, for example see Zhang, H.X., et al., Synthetic Communications, 17(11), (1987) 1299-1307. An excess of dimethylformamide dimethyl acetal is then added to the reaction which is allowed to stir for 2 to 6 hours. The solvent is removed under vacuum and the residue is purified by techniques well known to one skilled in the art to provide the compound defined by structure (2).

In Scheme A, step b, the 2'-hydroxyl group is oxidized to the ketone derivative defined by structure (3) by oxidation methods well known to one skilled in the art.

For example, approximately 1.5 equivalents of oxalyl chloride is dissolved in a suitable anhydrous organic solvent, such as methylene chloride, and cooled to about -75°C. To this solution is added 3 equivalents of methyl sulfoxide dropwise, maintaining the temperature below -55°C. An equivalent of the product defined by structure (2) is dissolved in a suitable amount of anhydrous organic solvent, such as methylene chloride, and added slowly to the reaction with stirring. After addition is complete the reaction is stirred for approximately 30 minutes at -75°C, an excess of a suitable organic base, such as triethylamine, is added and the reaction is allowed to warm to room temperature. The ketone derivative (3) is then isolated and purified by techniques well known to one skilled in the art. For example, silica gel chromatography followed by recrystallization from a suitable organic solvent or solvent mixture, such as 10% chloroform/hexane provides the ketone derivative (3).

In Scheme A, step c, the ketone derivative (3) can be olefinated to yield the corresponding exocyclic fluorovinyl sulfone (4) by reaction with a phosphorus ylide which can be prepared according to procedures which are well known and appreciated in the art of chemistry as described by March ["Advanced Organic Chemistry: Reactions, Mechanisms and Structure", McGraw-Hill Book Company, 2nd Ed., 1977, 864-872].

More specifically, olefination may be performed by reacting the appropriately substituted ketone derivative (3) with a suitable phosphonate ylide of formula (X)₂OP=CF(SO₂Ar) through a modification of the Wittig reaction as described by Wadsworth et al. [J. Am. Chem. Soc. 83 (1961), 1733]. For example, the appropriately substituted phosphonate of formula (X)₂OPCHF(SO₂Ar) is dissolved in a suitable anhydrous organic solvent and cooled to approximately -70°C. A suitable anhydrous organic solvent includes hydrocarbon solvents, dialkyl ethers, C₁-C₆ alkanes, tetrahydrofuran and the like. The preferred anhydrous organic solvent is tetrahydrofuran. An equivalent of a strong base is added slowly to produce the ylide. A wide variety of bases can be used including alkoxides and organometallics, such as alkyllithium, lithium dialkylamide, sodium hydride and the like. The preferred bases are potassium tert-butoxide and lithium bis(trimethylsilyl)amide. After approximately one hour an equivalent of the appropriately substituted ketone derivative (3) is added to the phosphonate ylide at approximately -60°C followed by warming to about 0°C for about 30 minutes and then warmed to room temperature for approximately 2.5 hours. The exocyclic fluorovinyl sulfone (4), is then isolated and purified by techniques well known to one skilled in the art. For example, the reaction is quenched with saturated ammonium chloride and the aqueous layer is extracted with a suitable organic solvent, such as diethyl ether. The organic phase is dried over a suitable drying agent, such as anhydrous magnesium sulfate, filtered and concentrated under vacuum. The crude material is filtered through silica gel with a suitable organic solvent, such as ethyl acetate to provide the exocyclic fluorovinyl sulfone (4).

In Scheme A, step d, deprotection of the exocyclic fluorovinyl sulfone (4) by removal of the nitrogen blocking group to provide the appropriately substituted deprotected amine derivative (5) is performed by dissolving the protected compound in an organic solvent, such as dioxane followed by treatment with an excess of a suitable base. A suitable base is one that is capable of removing the nitrogen blocking group without removing the silyl protecting group at the 3', 5' positions. Examples of suitable bases are ammonium hydroxide, ammonia, methylamine and the like. The preferred suitable base is ammonium hydroxide. The reaction is stirred for about 8 to 24 hours at room temperature and the deprotected amine (5) is isolated and purified by techniques well known to one skilled in the art. For example, the solvent is removed under vacuum azeotroping off the water with addition of ethanol and the crude material is purified by flash chromatography using a suitable solvent mixture, such as 5% hexane/ethyl acetate to provide the deprotected amine (5).

An alternative procedure for preparation of the deprotected amine (5) is performed by dissolving the appropriately substituted exocyclic fluorovinyl sulfone (4) in an organic solvent, such as ethyl acetate and treating with one equivalent of concentrated ammonium hydroxide for approximately 2 hours at room temperature. The deprotected amine (5) is isolated and purified by techniques well known to one skilled in the art. For example, the solvent is removed under vacuum azeotroping off the water with addition of ethanol and the crude material is purified by flash chromatography using a suitable solvent mixture, such as 5% hexane/ethyl acetate to provide the deprotected amine (5).

In Scheme A, step e, stannylation of the deprotected amine (5) utilizing procedures which are known to one of ordinary skill in the art as described by McCarthy et al. [J. Am. Chem. Soc., 113 (1991), 7439] provides the exocyclic (fluorovinyl)stannane of structure (6). For example, the deprotected amine (5) is dissolved in a suitable organic solvent, such as benzene or cyclohexane and treated with an excess of a suitable stannylating reagent of the formula (R)₃SnH. Suitable stannylating reagents are tributyltin hydride, triethyltin hydride, trimethyltin hydride, triphenyltin hydride and the like. The preferred stannylating reagent is tributyltin hydride. The reaction is then initiated by employing a suitable initiator. Suitable initiators are azobisisobutyronitiile (AIBN), UV light, heat and the like. The preferred suitable initiator is azobisisobutyronitrile (AIBN). A catalytic amount of AIBN is added and the reaction is heated at about 60 to 80°C for about 18 to 20 hours. Additional AIBN may be added as required to convert all the starting material to product. The additional AIBN can be added portionwise directly or as a solution in tetrahydrofuran during the course of the reaction. The additional amount of AIBN required can be readily determined by one of ordinary skill in the art by following the disappearance of starting material in the reaction utilizing techniques well known in the art, such as HPLC or thin layer chromatography. The product is then isolated and purified by techniques well known to one skilled in the art to provide the exocyclic (fluorovinyl)stannane defined by structure (6). For example, the reaction is concentrated under vacuum and the residue is purified by flash chromatography using a suitable solvent mixture, such as 4% to 6% methanol/methylene chloride to provide the exocyclic (fluorovinyl)stannane (6).

In Scheme A, step f, the exocyclic (fluorovinyl)stannane (6) can be sequentially converted to the exocyclic fluorovinyl derivative of Formula I by first destannylating with a protolysis agent under mild conditions in the absence of a fluoride ion source. A suitable protolysis agent will substitute a proton for the stannane substituent. Examples of a protolysis agent are ammonia/methanol, silica gel and the like. The protected exocyclic vinylfluoride is then deprotected by treatment with a suitable acid, such as aqueous hydrochloric acid or a fluoride ion source to provide the exocyclic vinylfluoride of Formula I. Examples of a fluorite ion source are sodium fluoride, potassium fluoride, cesium fluoride, tetrabutylammonium fluoride, ammonium fluoride and the like. The preferred fluoride ion source is potassium fluoride.

For example, in Scheme A, step f, the exocyclic (fluorovinyl)stannane (6) is combined with excess silica gel as described by Stork et al. [J. Am. Chem. Soc. 109 (1987), 2829] in a suitable organic solvent, such as methanol and allowed to stir until removal of the tributyltin is complete. The protected exocyclic vinylfluoride is then isolated and purified by techniques well known to one skilled in the art, such as flash chromatography. This product is then treated with an excess of a fluoride ion source, such as tetrabutylammonium fluoride, in a suitable organic solvent, such as methanol and allowed to stir until the deprotection is complete. The product is then isolated and purified by techniques well known to one of ordinary skill in the art to provide the exocyclic fluorovinyl compound defined by Formula I. For example, the reaction is concentrated under vacuum and purified by flash chromatography using a suitable solvent mixture, such as 50% ethyl acetate/hexane followed by 10% to 20% methanol/ethyl acetate. Recrystallization from methanol/ethyl acetate provides the compound of Formula I.

In Scheme A, step f, the exocyclic (fluorovinyl)stannane (6) can also be destannylated and deprotected concomitantly by reacting it with a protolysis agent and a fluoride ion source or a suitable acid to provide the exocyclic vinylfluoride of Formula I.

For example, in Scheme A, step f, the exocyclic (fluorovinyl)stannane (6) is dissolved in a suitable organic solvent, such as methanol, treated with a protolysis agent such as potassium fluoride (Kf may be in the dihydrate form), which also acts as a fluoride ion source and the reaction is heated at about 45 to 65°C for approximately 24 to 48 hours. After cooling, the solvent is partially concentrated and excess silica gel is added. The remaining solvent is removed and the product is isolated and purified by techniques well known to one of ordinary skill in the art to provide the compound defined by Formula I. For example, the reaction is concentrated under vacuum and purified by flash chromatography using a suitable solvent mixture, such as 50% ethyl acetate/hexane followed by 10% to 20% methanol/ethyl acetate.
Recrystallization from methanol/ethyl acetate provides the compound of Formula I.

Of course one skilled in the art would understand that the stepwise synthesis as presented in Scheme A is not limited to the particular sequence of steps as presented.

For example, in Scheme A, step c the olefination reaction may be performed subsequent to the deprotection reaction performed in step d.

An additional general synthetic process for preparation of compounds of formulas I and Ia is set forth in Scheme AI. All the substituents, unless otherwise indicated, are as previously defined. The reagents and starting materials for use in this process are readily available to one of ordinary skill in the art.

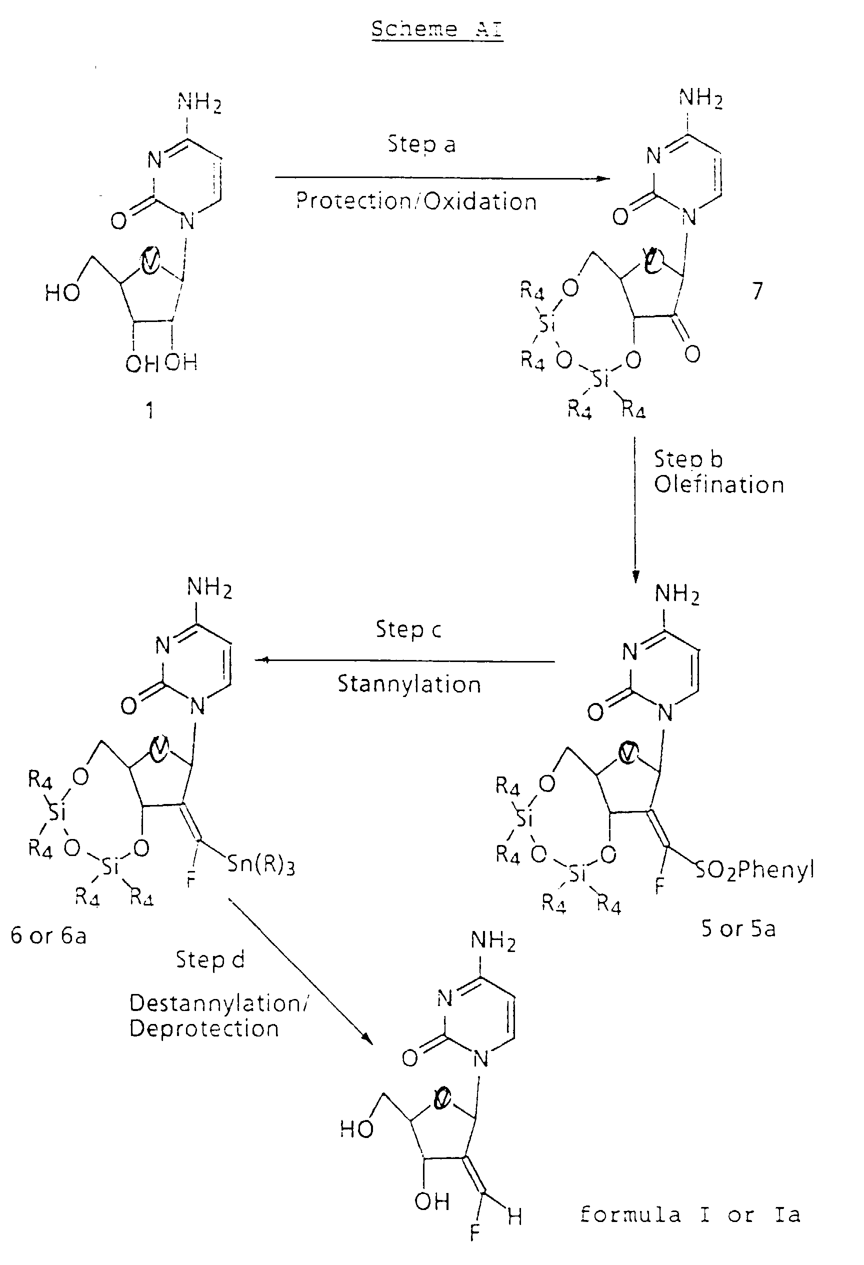

In Scheme AI step a, the 3' and 5' hydroxyls of the cytidine derivative of structure (1) are protected as the 1,3-(1,1,3,3-tetraalkyldisiloxanylidene) derivative and the 2' hydroxyl is oxidized to the ketone derivative as described by structure (7). For example, cytidine is combined with an excess of 1,3-dichloro-1,1,3,3-tetraalkyldisiloxane in a suitable dry organic solvent, such as pyridine under an inert atmosphere, such as nitrogen. The slurry is stirred at room temperature for about 5 to 24 hours and then cooled to about -17 to 5°C. Approximately 7 equivalents of dry triethylamine are added over a time period of about 1 hour followed by approximately 10 to 11 equivalents of dry dimethyl sulfoxide. Approximately 3 equivalents of SO₃-pyridine complex are added and the mixture is stirred at about -5 to 5°C for about 10 to 20 hours. The reaction is then poured into a mixture of ethyl acetate/water (2:1 by weight) which has been cooled to about 5°C. The original reactor is rinsed with a mixture of ethyl acetate/water (1.2:1.0 by weight) which is added to the previously quenched reaction mixture. Approximately 0.8 equivalents of "OXONE" (potassium peroxymonosulfate) are added to the mixture to oxidize dimethyl sulfide side-product, while maintaining the internal temperature of the reaction mixture below 15°C. The reaction is stirred for about 0.2 hours and then it is filtered to remove salts. The filter cake is rinsed with a suitable organic solvent, such as ethyl acetate. The product is then isolated by techniques well known in the art. For example, the filtrate phases are then separated and the organic phase is rinsed with water. The organic phase is substantially concentrated under vacuum and toluene is added. Again the mixture is substantially concentrated under vacuum. The toluene addition concentration procedure is continued until the distillate is water free. Toluene is again added, the mixture is cooled to about 15°C and then it is filtered. The filter cake is rinsed with toluene and dried at about 35°C under a flow of nitrogen to provide the 3',5'-protected-2'-keto derivative (7) as a white solid.

In Scheme AI step b, the 3',5'-protected-2'-keto derivative (7) is subjected to an olefination reaction to provide the (Z)-exocyclic fluorovinyl sulfone described by structure (5) following the procedure described previously in Scheme A step c. More specifically, the olefination reaction can be performed to stereoselectively produce the (Z)-isomer of the exocyclic fluorovinyl sulfone (5a). For example, approximately 1.05 equivalents of diethyl-1-fluoro-1-phenylsulfonylmethanephosphonate and a dry organic solvent, such as tetrahydrofuran are combined under an inert atmosphere such as nitrogen. The mixture is cooled to about -40°C and an equivalent of ketone derivative (7) is added to the slurry with stirring. The mixture is then cooled to about -50°C and approximately 1.03 equivalents of a potassium t-butoxide solution (about 20% in tetrahydrofuran) is added dropwise over a period of about 3 hours. After the addition is complete the reaction is allowed to warm to approximately -15°C over about 3 hours. Additional tetrahydrofuran may be added to aid in stirring as the reaction may thicken. The product is then isolated by techniques well known in the art. For example, the reaction can be quenched by vacuum transferring to an aqueous ammonium chloride solution at room temperature. The mixture is allowed to stir for about 30 minutes. The phases are then allowed to separate. The organic phase containing the (Z)-exocyclic fluorovinyl sulfone (5a) is separated from the aqueous phase and is carried on to step c in Scheme AI.

In Scheme AI step c, the exocyclic fluorovinyl sulfone (5) or the (Z)-exocyclic fluorovinyl sulfone (5a) is subjected to a stannylation reaction to provide the exocyclic (fluorovinyl)stannane or the (Z)-exocyclic (fluorovinyl)stannane described by structures (6) and (6a) under conditions generally described previously in Scheme A step e.

In Scheme AI step d, the exocyclic (fluorovinyl)stannane (6) or the (Z)-exocyclic (fluorovinyl)stannane (6a) can be sequentially or concomitantly destannylated and deprotected to provide the the exocyclic vinylfluoride of Formula I or the (E)-exocyclic vinylfluoride of Formula Ia under conditions generally described previously in Scheme A step f.

The appropriately substituted phosphonate of formula (X)₂OPCHF(SO₂Ar) required for preparation of the phosphonate ylide for reaction in Scheme A in step c and Scheme AI in step b can be obtained by a variety of methods described by Schemes B, C and D.

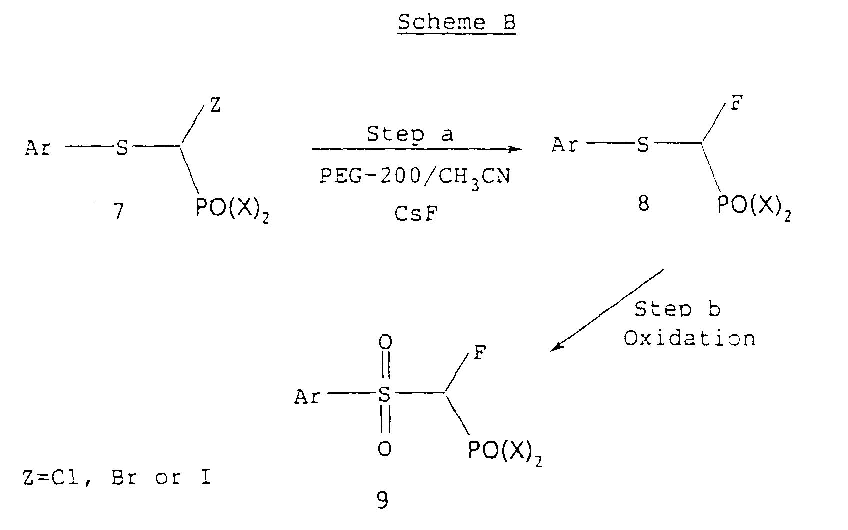

For example in Scheme B, step a, the compound defined by structure (7) is dissolved in a mixture of poly(ethylene glycol):acetonitrile in a ratio of approximately 1:2. A suitable molecular weight range for the poly(ethylene glycol) is between 100 and 400 g/mol. An excess of a fluoride ion source, such as cesium fluoride is added and the reaction is heated to approximately 80°C for 1 to 24 hours. The reaction is then diluted with water and the product extracted with a suitable organic solvent such as chloroform to provide after drying and concentrating under vacuum the product defined by structure (8). This is then oxidized by techniques well known to one skilled in the art. For example, treatment of compound (8) with potassium peroxymonosulfate in a suitable organic solvent, such as aqueous methanol to provides the appropriately substituted phosphonate defined by structure (9).

The appropriately substituted phosphonate defined by structure (9) can be obtained by another method as described in Scheme C.

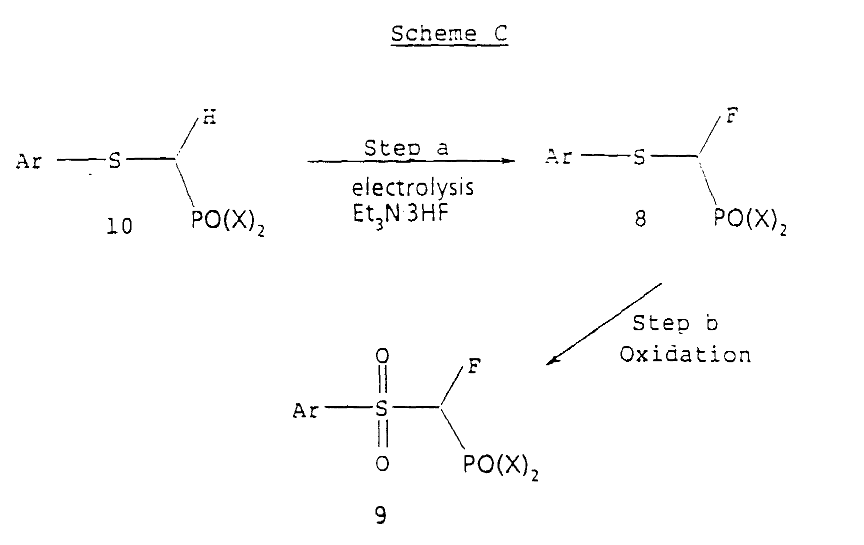

In Scheme C, step a, the compound defined by structure (10) is dissolved in a suitable organic solvent, such as tetrahydrofuran, treated with excess triethylamine trihydrofluoride and the solution is cooled to approximately -78°C. The solution is then subjected to a controlled potential electrolysis for about 3 to 10 hours to effectuate anodic monofluorination following generally the procedure of Matsue et al. [J. Chem. Soc., Chem. Commun. (1991), 1028]. The product defined by structure (8) is then isolated and oxidized in step b as described in Scheme B to provide the appropriately substituted phosphonate defined by structure (9).

Additionally, the appropriately substituted phosphonate defined by structure (9) may be prepared in situ as the ylide defined by the formula (X)₂OP=CF(SO₂Ar) as shown in Scheme D.

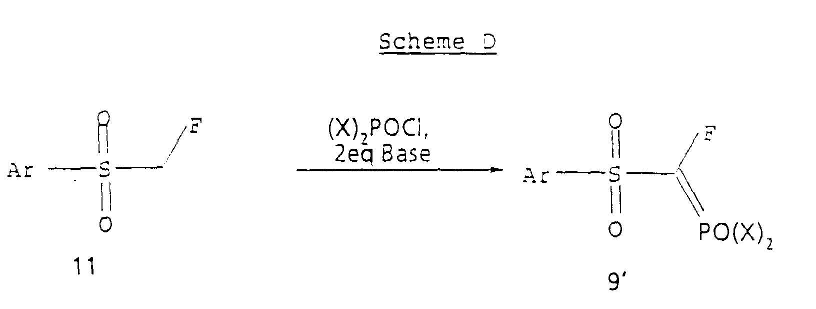

In Scheme D, the appropriately substituted sulfone, such as fluoromethylphenyl sulfone, which can be prepared according to McCarthy et al. [Tetrahedron Lett. 31 (1990), 5449], is dissolved in a suitable anhydrous organic solvent, such as tetrahydrofuran, cooled to approximately -70°C and treated with an equivalent of a dialkyl chlorophosphate, such as diethyl chlorophosphate, defined by the formula (X)₂POCl. The solution is then treated slowly with 2 equivalents of a strong base, such as lithium bis(trimethylsilyl)amide. After addition is complete the reaction is stirred at approximately -65°C for about 1 hour to provide the ylide defined by structure (9').

The following examples present typical syntheses as described by Schemes A, B, C and D. These examples are understood to be illustrative only and are not intended to limit the scope of the invention in any way. As used in the following examples, the following terms have the meanings indicated: "g" refers to grams, "mmol" refers to millimoles, "mL" refers to milliliters, "°C" refers to degrees Celsius, "TLC" refers to thin layer chromatography, "mg" refers to milligrams, "µL" refers to microliters and "δ" refers to parts per million downfield from tetramethlysilane.

### Example 1

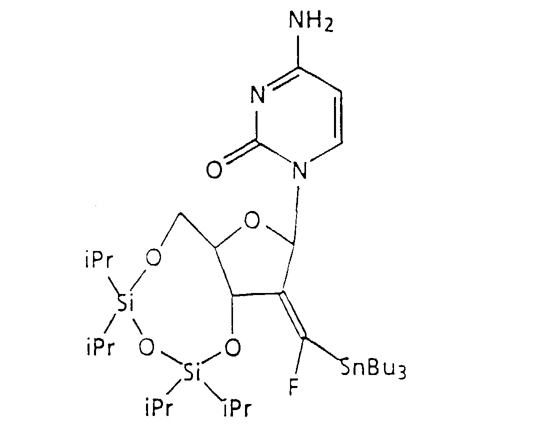

### Preparation of (Z)-2'-deoxy-2'-[fluoro(tributylstannyl)methylene]-3',5'-O-[1,1,3,3-tetrakis(1-methylethyl)-1,3-disiloxanediyl]-cytidine.

A solution of (Z)-2'-[fluoro(phenylsulfonyl)methylene]-3',5'-O-[1,1,3,3-tetrakis(l-methylethyl)-1,3-disiloxanediyl]-cytidine (1.327 Kg) is checked with potassium iodide-starch paper to verify the absence of peroxides and is then concentrated under vacuum (35°C to 36°C) to provide a cloudy yellow oil that is 44% to 49% by weight of the above starting material. The material is subjected to azeotropic drying with cyclohexane at 20°C to 40°C and 90 to 120 torr. Approximately 9 to 11 kg of cyclohexane is used to effect the drying, affording a final cyclohexane solution that is 46% to 49% by weight of the above starting material. This solution is transferred to a 22 liter round bottomed flask. Additional cyclohexane (0.55 to 1.2 Kg) is used to rinse any cyclohexane residual to the 22 liter round bottom flask. To this solution is added tributyltin hydride (3.6 to 4.0 Kg, 2.5 to 2.7 equivalents) and azoisobutyronitrile (44 to 55 g, AIBN) at room temperature. The stirred reaction mixture is placed under nitrogen and heated to 60°C to 65°C. The reaction mixture is allowed to stir at this temperature for 18 to 20 hours, during which time a solution of AIBN (240 to 300 g) in tetrahydrofuran (2.5 to 3.4 Kg) is added in a slow stream. The reaction mixture is cooled to room temperature and transferred to a 50 liter bottom drained flask. Tetrahydrofuran (9 to 10 Kg) is added. The resulting solution is extracted with two portions of one normal aqueous potassium hydroxide (13 to 15 Kg per extraction) and the resulting lower aqueous phases are removed from the 50 liter flask. To the product solution in the 50 liter flask is added tetrahydrofuran (4 to 5 Kg) and the resulting solution is extracted with water (13 to 15 Kg). The lower aqueous phase is removed and the product solution is concentrated under vacuum at 40°C to 45°C to provide the title compound as a viscous orange/brown oil (5.8 to 6.9 Kg).

### Example 2

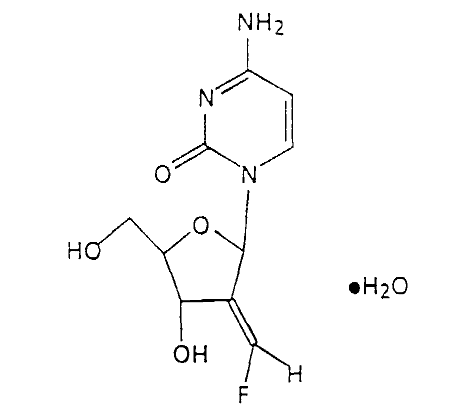

### Preparation of (E)-2'-deoxy-2'-(fluoromethylene)cytidine monohydrate.

The (2'Z)-2'-deoxy-2'-[fluoro(tributylstannyl)methylene]-3',5'-O-[1,1,3,3-tetrakis(l-methylethyl)-1,3-disiloxanediyl]-cytidine (6.39 Kg) prepared in example 1 is dissolved in methanol (9.24 Kg). 50% wt potassium fluoride dihydrate (4.03 Kg diluted with 1.22 Kg of water) is added with stirring. The reaction is heated to 45°C for 48 hours. The reaction is then concentrated under vacuum at a bath temperature cf 30°C. The concentrate is partitioned between water (4.0 Kg) and ethyl acetate (4.0 Kg). The lower aqueous layer is collected and saved. The upper organic layer containing tributyltin compounds and tetraisopropylsiloxyl compounds is discarded. The middle emulsion layer is filtered and the filtercake is rinsed with water (1.0 Kg) and ethyl acetate (1.0 Kg). The filtrate is allowed to separate and the lower aqueous layer is collected. The aqueous layers are then combined and rinsed with ethyl acetate (2 Kg). Diatomaceous earth (0.05 Kg) is added to the combined aqueous layers which is then vacuum filtered. The filtrate is concentrated under vacuum at a bath temperature of 30°C. Methanol (3.0 Kg) is added to the concentrate and the mixture is again concentrated under vacuum. The residue is then dissolved in methanol (5.0 Kg) and silica gel 60 (4.0 Kg, 100-200 mesh) is added. The mixture is evaporated with a continuous addition of isopropanol to maintain the original volume nearly constant. After approximately 20 Kg of distillate is collected, the slurry is added to a column containing silica gel (2.0 Kg) that has been preconditioned with isopropanol. The column is eluted with isopropanol until no product is detected in the fractions. The fractions are combined and concentrated to approximately 4 liters total volume. The slurry is filtered and the filtercake rinsed with isopropanol (1.0 Kg). The filtercake is air dried to a constant weight (0.83 Kg) to provide the crude anhydrous form of the title compound. This material is combined with similar batches and the total amount (3.82 Kg) is dissolved in water (22 Kg) and polish filtered. The filtrate is concentrated to 8-10 liters total volume under vacuum at a bath temperature of 30°C. The resulting slurry is cooled at 5°C for 2 hours. The product is collected by vacuum filtration and rinsed with cold water (1.8 Kg). The filtercake is air dried to a constant weight (3.31 Kg, 99.8% pure by HPLC, corrected for water) to provide the title compound.

### Example 3

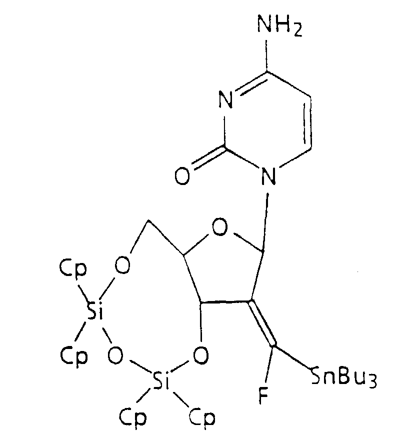

### Preparation of(Z)-2'-deoxy-2'-[fluoro(tributylstannyl)methylene]-3',5'-O-[1,1,3,3-tetrakis(cyclopentyl)-1,3-disiloxanediyl]-cytidine.

The title compound can be prepared from (Z)-2'-[fluoro(phenylsulfonyl)methylene]-3',5'-O-[1,1,3,3-tetrakis(cyclopentyl)-1,3-disiloxanediyl]-cytidine (1.327 Kg) tributyl tin hydride (2.5 to 2.7 equivalents) and azoisobutyronitrile (44 to 55 g, AIBN) in a manner analogous to the procedure described in example 1.

Following the respective procedures, the monohydrate of the final product prepared in example 2, can be prepared in an (Z)-2'-deoxy-2'-[fluoro(tributylstannyl)methylene]-3',5'-O-[1,1,3,3-tetrakis(cyclopentyl)-1,3-disiloxanediyl]-cytidine prepared in example 3.

## Claims

1. A compound of the formula 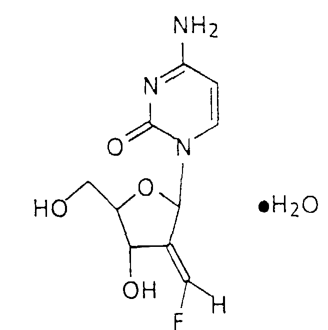 which is (E)-2'-deoxy-2'-(fluoromethylene)cytidine monohydrate.

2. A pharmaceutical composition comprising a compound as defined in claim 1.

3. A method for the preparation of the compound as defined in claim 1 comprising the steps of:
(a) reacting a compound of the formula 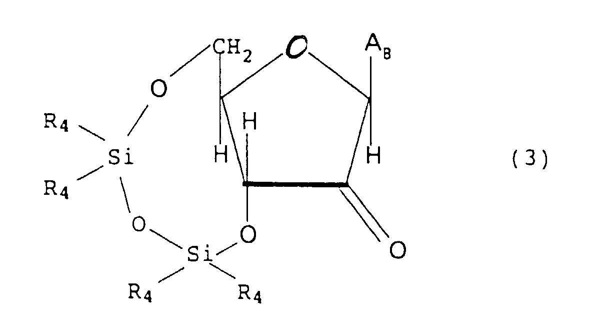 wherein R₄ is C₂-C₇ alkyl or C₅-C₇ cycloalkyl and A_{B} is a radical of the formula 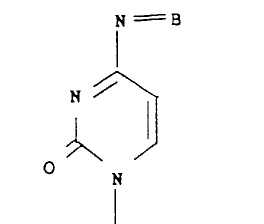 and B is a suitable nitrogen blocking group of the formula CR₁NR₂R₃ wherein R₁ is a hydrogen or C₁-C₄ alkyl and R₂ and R₃ are independently C₁-C₄ alkyl, with a suitable phosphonate ylid of the formula (X)₂OP=CF(SO₂Ar) wherein Ar is an aryl group and X is a phenoxy or C₁-C₄ alkoxy to produce an exocyclic fluorovinyl sulfone;
(b) reacting the exocyclic fluorovinyl sulfone with a suitable base or suitable weak acid to produce a deprotected amine;
(c) reacting the deprotected amine with a stannylating reagent of the formula (R)₃SnH wherein R is aryl or C₁-C₄ alkyl to produce an exocyclic (fluorovinyl)stannane;
(d) reacting the exocyclic (fluorovinyl)stannane with a protolysis agent and, either concomitantly or sequentially, reacting the silyl protecting group with a suitable acid or a fluoride ion source.

4. A method for the preparation of the compound as defined in Claim 1 comprising the steps of:
(a) reacting a compound of the formula 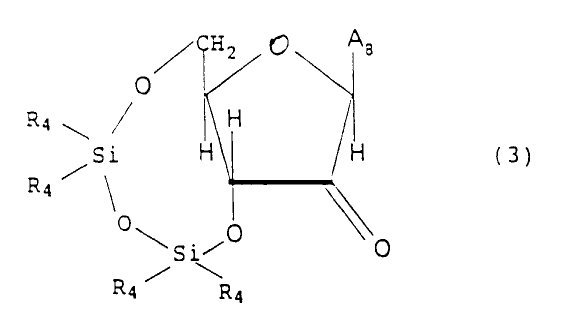 wherein R₄ is C₂-C₇ alkyl or C₅-C₇ cycloalkyl and A_{B} is a radical of the formula 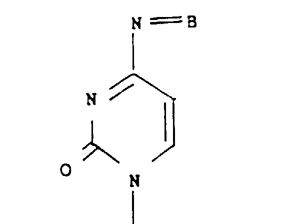 and B is a suitable nitrogen blocking group of the formula CR₁NR₂R₃ wherein R₁ is a hydrogen or C₁-C₄ alkyl and R₂ and R₃ are independently C₁-C₄ alkyl, with a suitable base or a suitable weak acid to produce a deprotected amine;
(b) reacting the deprotected amine with a suitable phosphonate ylid of the formula (X)₂OP=CF(SO₂Ar) wherein Ar is an aryl group and X is a phenoxy or C₁-C₄ alkoxy to produce an exocyclic fluorovinyl sulfone;
(c) reacting the exocyclic fluorovinyl sulfone with a stannylating reagent of the formula (R)₃SnH wherein R is aryl or C₁-C₄ alkyl to produce an exocyclic (fluorovinyl)stannane;
(d) reacting the exocyclic (fluorovinyl)stannane with a protolysis agent and, either concomitantly or sequentially, reacting the silyl protecting group with a suitable acid or a fluoride ion source.

5. A method for the preparation of the compound as defined in claim 1, comprising the steps of:
(a) reacting a compound of the formula 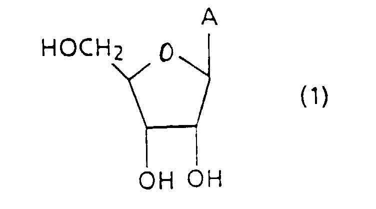 wherein A is a radical of the formula 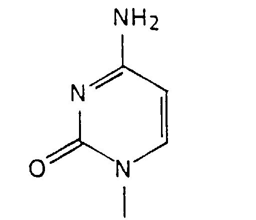 with excess 1,3-dichloro-1,1,3,3-tetraalkyldisiloxane and triethylamine followed by treatment with SO₃-pyridine complex to produce a 3',5'-protected-2'-keto derivative;
(b) reacting the 3',5'-protected-2'-keto derivative with a suitable phosphonate ylid of the formula (X)₂OP=CF(SO₂Ar) wherein Ar is an aryl group and X is a phenoxy or C₁-C₄ alkoxy to produce an exocyclic fluorovinyl sulfone;
(c) reacting the exocyclic fluorovinyl sulfone with a stannylating reagent of the formula (R)₃SnH wherein R is aryl or C₁-C₄ alkyl to produce an exocyclic (fluorovinyl)stannane;
(d) reacting the exocyclic (fluorovinyl)stannane with a protolysis agent and, either concomitantly or sequentially, reacting the silyl protecting group with a suitable acid or a fluoride ion source.

## Patentansprüche

1. Verbindung der Formel 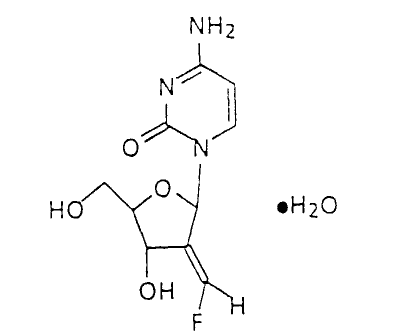 die (E)-2'-Desoxy-2'-(fluormethylen)cytidin-monohydrat ist.

2. Arzneimittel, das eine Verbindung, wie in Anspruch 1 definiert, umfaßt.

3. Verfahren zur Herstellung der in Anspruch 1 definierten Verbindung, umfassend die Schritte:
(a) Umsetzen einer Verbindung der Formel 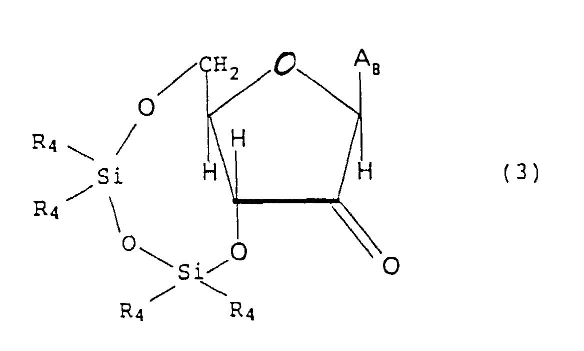 wobei R₄ ein C₂-C₇-Alkyl- oder C₅-C₇-Cycloalkylrest ist und A_{B} einen Rest der Formel 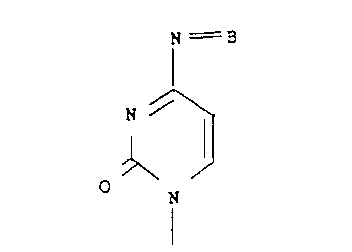 darstellt und B eine geeignete Stickstoffschutzgruppe der Formel CR₁NR₂R₃ bedeutet, wobei R₁ ein Wasserstoffatom oder ein C₁-C₄-Alkylrest ist und R₂ und R₃ unabhängig C₁-C₄-Alkylreste sind, mit einem geeigneten Phosphonatylid der Formel (X)₂OP=CF(SO₂Ar), wobei Ar ein Arylrest ist und X einen Phenoxy- oder C₁-C₄-Alkoxyrest darstellt, um ein exocyclisches Fluorvinylsulfon herzustellen;
(b) Umsetzen des exocyclischen Fluorvinylsulfons mit einer geeigneten Base oder einer geeigneten schwachen Säure, um ein ungeschütztes Amin herzustellen;
(c) Umsetzen des ungeschützten Amins mit einem Stannylierungsmittel der Formel (R)₃SnH, wobei R ein Aryl- oder C₁-C₄-Alkylrest ist, um ein exocyclisches (Fluorvinyl)stannan herzustellen;
(d) Umsetzen des exocyclischen (Fluorvinyl)stannans mit einem Protolysemittel und, entweder gleichzeitig oder nacheinander, Umsetzen der Silylschutzgruppe mit einer geeigneten Säure oder einer Fluoridionenquelle.

4. Verfahren zur Herstellung der in Anspruch 1 definierten Verbindung, umfassend die Schritte:
(a) Umsetzen einer Verbindung der Formel 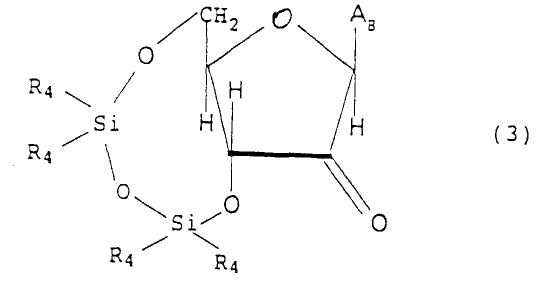 wobei R₄ ein C₂-C₇-Alkyl- oder C₅-C₇-Cycloalkylrest ist und A_{B} einen Rest der Formel 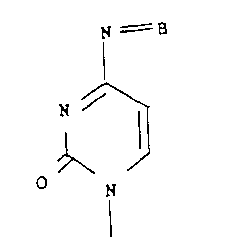 darstellt und B eine geeignete Stickstoffschutzgruppe der Formel CR₁NR₂R₃ bedeutet, wobei R₁ ein Wasserstoffatom oder ein C₁-C₄-Alkylrest ist und R₂ und R₃ unabhängig C₁-C₄-Alkylreste sind, mit einer geeigneten Base oder einer geeigneten schwachen Säure, um ein ungeschütztes Amin herzustellen;
(b) Umsetzen des ungeschützten Amins mit einem geeigneten Phosphonatylid der Formel (X)₂OP=CF(SO₂Ar), wobei Ar ein Arylrest ist und X einen Phenoxy- oder C₁-C₄-Alkoxyrest darstellt, um ein exocyclisches Fluorvinylsulfon herzustellen;
(c) Umsetzen des exocyclischen Fluorvinylsulfons mit einem Stannylierungsmittel der Formel (R)₃SnH, wobei R ein Aryl- oder C₁-C₄-Alkylrest ist, um ein exocyclisches (Fluorvinyl)stannan herzustellen;
(d) Umsetzen des exocyclischen (Fluorvinyl)stannans mit einem Protolysemittel und, entweder gleichzeitig oder nacheinander, Umsetzen der Silylschutzgruppe mit einer geeigneten Säure oder einer Fluoridionenquelle.

5. Verfahren zur Herstellung der in Anspruch 1 definierten Verbindung, umfassend die Schritte:
(a) Umsetzen einer Verbindung der Formel 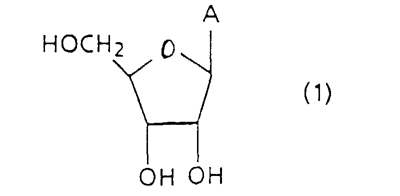 wobei A ein Rest der Formel 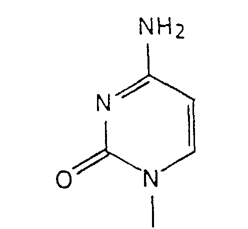 ist, mit einem Überschuß an 1,3-Dichlor-1,1,3,3-tetraalkyldisiloxan und Triethylamin und anschließende Umsetzung mit SO₃-Pyridin-Komplex, um ein 3',5'-geschütztes 2'-Ketoderivat herzustellen;
(b) Umsetzen des 3',5'-geschützten 2'-Ketoderivats mit einem geeigneten Phosphonatylid der Formel (X)₂OP=CF(SO₂Ar), wobei Ar ein Arylrest ist und X einen Phenoxy- oder C₁-C₄-Alkoxyrest darstellt, um ein exocyclisches Fluorvinylsulfon herzustellen;
(c) Umsetzen des exocyclischen (Fluorvinyl)sulfons mit einem Stannylierungsmittel der Formel (R)₃SnH, wobei R ein Aryl- oder C₁-C₄-Alkylrest ist, um ein exocyclisches (Fluorvinyl)stannan herzustellen;
(d) Umsetzen des exocyclischen (Fluorvinyl)stannans mit einem Protolysemittel und, entweder gleichzeitig oder nacheinander, Umsetzen der Silylschutzgruppe mit einer geeigneten Säure oder einer Fluoridionenquelle.

## Revendications

1. Composé de la formule 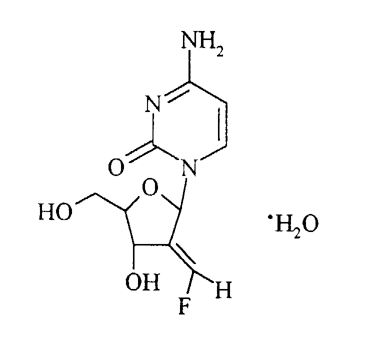 lequel est le monohydrate de (E)-2'-déoxy-2'-(fluorométhylène)cytidine.

2. Composition pharmaceutique comprenant un composé comme défini dans la revendication 1.

3. Procédé pour la préparation du composé selon la revendication 1 comprenant les étapes consistant :
(a) à faire réagir un composé de la formule 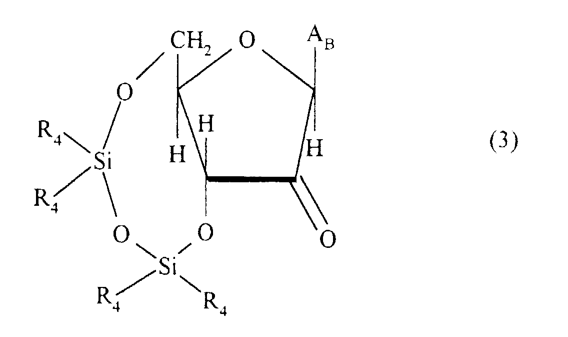 dans laquelle R₄ est un groupe alkyle en C₂-C₇ ou cycloalkyle en C₅-C₇ et A_{B} est un radical de la formule 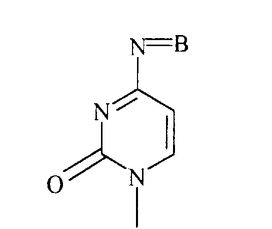 et B est un groupe approprié bloquant l'azote de la formule CR₁NR₂R₃ dans laquelle R₁ est un atome d'hydrogène ou un groupe alkyle en C₁-C₄ et R₂ et R₃ sont indépendamment un groupe alkyle en C₁-C₄ avec un ylide phosphanate approprié de la formule (X)₂OP=CF(SO₂Ar), dans laquelle Ar est un groupe aryle et X est un groupe phénoxy ou alcoxy en C₁-C₄ pour produire une fluorovinylsulfone exocyclique;
(b) à faire réagir la fluorovinylsulfone exocyclique avec une base appropriée ou un acide faible approprié pour produire une amine déprotégée;
(c) à faire réagir l'amine déprotégée avec un réactif de stannylation de la formule (R)₃SnH, dans laquelle R est un groupe aryle ou alkyle en C₁-C₄ pour produire un (fluorovinyl)stannane exocyclique;
(d) à faire réagir le (fluorovinyl)stannane exocyclique avec un agent de protolyse et à faire réagir soit de manière concomitante, soit successivement le groupe silyle protecteur avec un acide approprié ou une source d'ions fluorure.

4. Procédé pour la préparation du composé selon la revendication 1 comprenant les étapes consistant :
(a) à faire réagir un composé de la formule 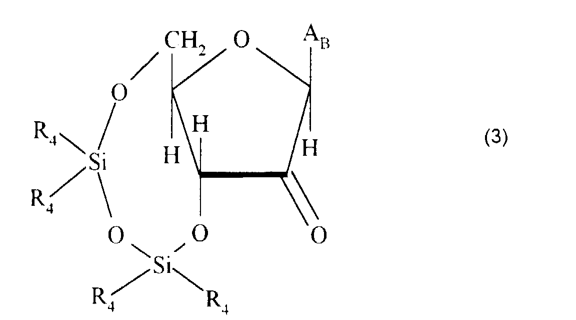 dans laquelle R₄ est un groupe alkyle en C₂-C₇ ou cycloalkyle en C₅-C₇ et A_{B} est un radical de la formule 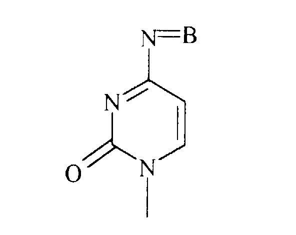 et B est un groupe approprié bloquant l'azote de la formule CR₁NR₂R₃ dans laquelle R₁ est un atome d'hydrogène ou un groupe alkyle en C₁-C₄ et R₂ et R₃ sont indépendamment un groupe alkyle en C₁-C₄ avec une base appropriée ou un acide faible approprié pour produire une amine déprotégée;
(b) à faire réagir l'amine déprotégée avec un ylide phosphanate approprié de la formule (X)₂OP=CF(SO₂Ar), dans laquelle Ar est un groupe aryle et X est un groupe phénoxy ou alcoxy en C₁-C₄ pour produire une fluorovinylsulfone exocyclique;
(c) à faire réagir la fluorovinylsulfone exocyclique avec un réactif de stannylation de la formule (R)₃SnH, dans laquelle R est un groupe aryle ou alkyle en C₁-C₄ pour produire un (fluorovinyl)stannane exocyclique,
(d) à faire réagir le (tluorovinyl)stannane exocyclique avec un agent de protolyse et à faire réagir soit de manière concomitante, soit successivement le groupe silyle protecteur avec un acide approprié ou une source d'ions fluorure.

5. Procédé pour la préparation du composé selon la revendication 1 comprenant les étapes consistant :
(a) à faire réagir un composé de la formule 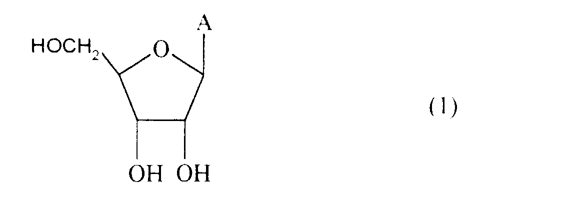 dans laquelle A est un radical de la formule 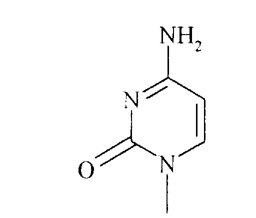 avec du 1,3-dichloro-1,1,3,3,-tétraalkyldisiloxane en excès et de la triéthylamine suivi par un traitement avec un complexe de SO₃-pyridine pour produire un dérivé 2'-céto 3',5'-protégé;
(b) à faire réagir le dérivé 2'-céto 3',5'-protégé avec un ylide phosphanate approprié de la formule (X)₂OP=CF(SO₂Ar), dans laquelle Ar est un groupe aryle et X est un groupe phénoxy ou alcoxy en C₁-C₄ pour produire une fluorovinylsulfone exocyclique;
(c) à faire réagir la fluorovinylsulfone exocyclique avec un réactif de stannylation de la formule (R)3SnH, dans laquelle R est un groupe aryle ou alkyle en C₁-C₄ pour produire un (fluorovinyl)stannane exocyclique;
(d) à faire réagir le (fluorovinyl)stannane exocyclique avec un agent de protolyse et à faire réagir soit de manière concomitante, soit successivement le groupe silyle protecteur avec un acide approprié ou une source d'ions fluorure.
